# NEUE EUROPÄISCHE PATENTSCHRIFT

(11) **EP 3 618 752 B2**
(45) Veröffentlichungstag und Bekanntmachung des Hinweises auf die Entscheidung über den Einspruch: **16.08.2023**
(45) Hinweis auf die Patenterteilung: 21.10.2020
(21) Anmeldenummer: 19731706.8
(22) Anmeldetag: 17.06.2019
(51) Int. Cl.: A61C 1/08

(54) **VERFAHREN ZUR KONSTRUKTION EINER BOHRSCHABLONE**
METHOD FOR DESIGNING A DRILLING TEMPLATE
PROCÉDÉ DE RÉALISATION D'UN GABARIT DE PERÇAGE

(30) Priorität: 22.06.2018 DE 102018210259
(43) Veröffentlichungstag der Anmeldung: 11.03.2020
(73) Patentinhaber: Sirona Dental Systems GmbH, 64625 Bensheim (DE); Dentsply Sirona Inc., York, PA 17401-2991 (US)
(72) Erfinder: SCHNEIDER, Sascha, 64367 Mühltal (DE); THIEL, Frank, 64372 Ober-Ramstadt (DE); SCHWOTZER, Axel, 64521 Gross-Gerau (DE)
(74) Vertreter: Özer, Alpdeniz
(86) Internationale Anmeldenummer: PCT/EP2019/065837
(87) Internationale Veröffentlichungsnummer: WO 2019/243233

(56) Entgegenhaltungen:
- WO-A1-2018/195554
- WO-A1-2019/067995
- US-A1- 2012 214 121

## Beschreibung

### Technisches Gebiet

Die Erfindung betrifft ein Verfahren zur Konstruktion einer Bohrschablone, wobei mittels einer dentalen Kamera oder eines Labor-Scanners eine Zahnsituation vermessen wird und ein 3D-Oberflächenmodell der Zahnsituation erzeugt wird und/oder mittels einer Röntgenvorrichtung oder einer MRT-Vorrichtung die Zahnsituation vermessen wird und ein Volumenmodell der Zahnsituation erzeugt wird.

### Stand der Technik

Aus dem Stand der Technik sind mehrere Verfahren zur Konstruktion von Bohrschablonen bekannt.

In der DE 199 52 962 A1 ist ein Verfahren zur Erstellung einer Bohrhilfe für ein Zahnimplantat offenbart. Dabei ist die Bohrhilfe mit Anlageflächen passend zu Okklusalflächen von Nachbarzähnen ausgestattet und wird zur exakten Positionierung in Bezug auf den Kieferabschnitt auf die Nachbarzähne aufgesetzt. In der Bohrhilfe ist ein Führungsloch vorgesehen, um einen Bohrer eines Handstücks zu führen. Die Position, die Abmessungen und die Ausrichtung des Führungsloches in der Bohrschablone sind durch die geplante Implantatbohrung vorgegeben.

Aus der DE 10 2009 008 790 B3 ist eine Bohrschablone mit einer Führungshülse zum Führen eines oralchirurgischen Bohrers offenbart. Der Bohrer kann auch bei minimalen Platzverhältnissen in die Führungshülse eingesetzt werden, wenn diese in ihrer Umfangsfläche ein bezüglich der Hülsenlängenachse schräg verlaufenden Schlitz aufweist. Eine solche Führungshülse erlaubt darüber hinaus eine nahezu optimale Führung des Bohrers in Richtung der geplanten Bohrachse.

DE 199 52 962 A1 offenbart ein Verfahren zur Erstellung einer Bohrhilfe für ein Zahnimplantat, wobei zunächst eine Röntgenaufnahme des Kiefers und danach eine dreidimensionale optische Vermessung der sichtbaren Oberfläche des Kiefers und der Zähne durchgeführt werden. Die Messdatensätze von der Röntgenaufnahme und der dreidimensionalen optischen Aufnahme werden miteinander korreliert. Anhand der vorhandenen Informationen, wie der Art und der Position des Implantats relativ zu den Nachbarzähnen, wird eine Schablone geplant und generiert, die auf den Nachbarzähnen aufliegt und dadurch die exakte Bohrung des Implantatführungslochs ermöglicht wird. Anhand der Röntgendaten kann das Implantat in bekannter Weise bestimmt und positioniert werden. Anhand der gewonnenen Informationen über die Oberflächenstruktur, d.h. die Okklusalflächen benachbarter Zähne, kann eine Implantationshilfe in Gestalt einer Bohrschablone mittels einer CAD/CAM-Einheit ausgeschliffen werden. Anhand der Messdaten ist eine CAD/CAM-Maschine in der Lage, die Bohrschablone mit dem Negativ der Okklusalflächen und einem Führungslauf für den Bohrer zu fertigen. Auf die Bohrschablone wird ein Anschlag übertragen, der die Bohrtiefe bestimmt.

WO 99/32045 A1 offenbart ein Verfahren zur Herstellung einer dentalen Bohrhilfe für Implantate. Zunächst wird unter Verwendung eines Kieferbildes mit Bezug auf eine Abdruckfläche ein dreidimensionales Rechnerbild modelliert. Danach werden die Lage und die Bohrtiefe der Bohrlöcher bestimmt und ein Satz von Implantatbohrlochkoordinaten in eine computergesteuerte Fertigungsmaschine eingespeist. Mittels eines Präzisionswerkzeugs wird im Bohrkörper für jeden der zuvor eingespeisten Bohrlochkoordinatensätze ein Bohrführungssockel vorbereitet mit einer entsprechend der anhand des Kieferabschnitts ermittelten Bohrlochposition und Bohrlochorientierung.

Aus der EP 1 502 556 A2 ist eine Bohrschablone sowie ein Verfahren zur Herstellung dieser Bohrschablone bekannt, wobei die Bohrschablone mindestens ein Durchgangsloch zur Durchführung des Bohrers aufweist, in das mindestens eine erste Führungshülse einsetzbar ist. Die Führungshülse weist in einem unteren Bereich einen Außendurchmesser auf, der im Wesentlichen dem Innendurchmesser des Durchgangslochs entspricht. In einem oberen Bereich weist die Führungshülse einen größeren Außendurchmesser auf, so dass sich der obere Abschnitt auf dem das Durchgangsloch umgebenden Rand der Bohrschablone abstützt.

US 2012/0214121 A1 offenbart ein Verfahren zum Erzeugen eines Zahnmodells, wobei CT-Bilddaten mit optischen Oberflächendaten registriert werden. Das Zahnmodell wird auf der Grundlage von segmentierten anatomischen Strukturen, wie Wurzeln, Knochen, erstellt.

WO 2018/195554 A1 offenbart ein System und ein Verfahren zur Herstellung eines Zahnmodells, wobei die einzelnen Zähne eines Zahnmodells identifiziert werden und Zielpositionen von Komponentenmodellen bestimmt werden.

WO 2019/067995 A1 offenbart ein Verfahren zur Inspektion einer zahnärztlichen Vorrichtung auf Herstellungsfehler, wobei gewünschte Eigenschaften der zahnärztlichen Vorrichtung mit den tatsächlichen Eigenschaften verglichen werden.

Ein Nachteil der genannten Verfahren zur Konstruktion von Bohrschablonen besteht darin, dass diese auf eine aufwendige Art und Weise mittels des Benutzers virtuell oder durch Verwendung von Abdrücken konstruiert werden. Durch Messungenauigkeiten oder durch Konstruktionsfehler des Benutzers können dann Passungenauigkeiten der Bohrschablone entstehen. Eine ungenaue Bohrschablone kann folglich zu einer falschen Implantatbohrung abweichend von der geplanten Implantatbohrung führen.

Die Aufgabe der vorliegenden Erfindung besteht daher darin, ein Verfahren zur Konstruktion einer Bohrschablone bereitzustellen, welches zeitsparend ein passendes 3D-Modell der Bohrschablone konstruiert.

### Darstellung der Erfindung

Die Erfindung betrifft ein Verfahren zur Konstruktion einer Bohrschablone, wobei mittels einer 3D-Oberflächenvermessungsvorrichtung, wie einer dentalen Kamera oder eines Labor-Scanners, eine Zahnsituation vermessen wird und ein 3D-Oberflächenmodell der Zahnsituation erzeugt wird und mittels einer Röntgenvorrichtung oder einer MRT-Vorrichtung die Zahnsituation vermessen wird und ein Volumenmodell der Zahnsituation erzeugt wird. Dabei wird ein künstliches neuronales Netz für maschinelles Lernen (engl. Convolutional Neural Network; CNN) auf das 3D-Oberflächenmodell der Zahnsituation und das Volumenmodell der Zahnsituation angewendet und automatisch ein fertig konstruiertes 3D-Modell der Bohrschablone erzeugt.

Die zahnmedizinische Bohrschablone kann eine beliebige Bohrschablone, wie eine durch die Nachbarzähne getragene Bohrschablone für minimalinvasive Chirurgie sowie eine durch den Kieferknochen getragene Bohrschablone für die sogenannte Open-Flap-Chirurgie sein. Dabei wird also die Bohrschablone durch die übrigen Nachbarzähne des jeweiligen Kiefers getragen oder mittels Schrauben am Kieferknochen festgeschraubt.

Die Bohrschablone dient zur Führung eines Bohrers, um eine geplante Implantatbohrung zum Einsetzen eines Implantats, wie in einer Implantatplanung errechnet, durchzuführen. An einer Durchgangsöffnung kann eine Hülse in die Durchgangsöffnung gesteckt werden, wobei die innere Fläche der Hülse zur Führung des Bohrers dient. Bei der Implantatplanung kann das Volumenmodell des Kiefers und eine dreidimensionale optische Vermessung der sichtbaren Oberfläche des Kiefers und der Zähne in Form eines 3D-Oberflächenmodells verwendet werden, um einen Implantattyp und die Implantatlage relativ zum Kiefer virtuell festzulegen. Insbesondere werden die genaue Lage, der relative Winkel zum Kiefer und die Tiefe der Implantatbohrungen zum Einsetzen der Implantate geplant. Die Bohrschablone ist zur Durchführung einer oder mehrerer Implantatbohrungen vorgesehen, die auch windschief zueinander angeordnet sein können.

Dabei werden insbesondere anatomische Strukturen innerhalb des Kiefers, wie Zahnwurzeln, Zahnnerven und Verlauf und Dicke des Kieferknochens berücksichtigt.

In Abhängigkeit vom gewählten Implantat und der Implantatlage wird anschließend die Bohrschablone in Abhängigkeit vom 3D-Oberflächenmodell der Zahnsituation und des Volumenmodells konstruiert.

Das neuronale Netz kann also automatisch die folgenden Schritte durchführen: die Auswahl eines passenden Implantattyps; die Anordnung des Implantats relativ zum Kiefer unter Berücksichtigung anatomische Strukturen, wie Zahnwurzeln, Zahnnerven und Kieferknochen; Konstruktionen eines 3D-Modells der Bohrschablone umfassend mindestens ein Führungsloch für mindestens eine Implantatbohrung für das einzusetzende Implantat unter Berücksichtigung des 3D-Oberflächenmodells der Zahnsituation und des Volumenmodells.

Eine zahngetragene Bohrschablone kann einen Abdruck der Nachbarzähne der Implantationsstelle zur Abstützung auf den Nachbarzähnen umfassen. Eine Führungsbohrung kann aufgeweitet gestaltet sein. Die Führungsbohrung kann insbesondere kegelförmig mit einer ovalen oder kreisförmigen Grundfläche ausgebildet sein. Die Hülse kann dabei ein zylindrisches Hülsenteil aufweisen, das in die Durchgangsöffnung hineinpasst.

Bei Bohrschablonen, die mittels Schrauben am Kieferknochen fixiert werden, weist die Bohrschablone zusätzlich zu den Führungsbohrungen weitere Öffnungen für die Schrauben auf.

Eine Bohrschablone mit Hülsen kann unterschiedliche Hülsen für die gleiche Führungsbohrung mit unterschiedlichen Durchmessern aufweisen, um zunächst eine Bohrung mit dem kleinsten Durchmesser durchzuführen und anschließend durch das Ersetzen der Hülse und des jeweiligen Bohrers weitere Bohrungen mit steigendem Durchmesser durchzuführen, bis der gewünschte Durchmesser an der Implantatbohrung erreicht ist.

Die Bohrschablone kann nach der Durchführung der Implantatbohrung verwendet werden, um ein geführtes Eindrehen des Implantats in die Implantatbohrung durchzuführen.

Die Bohrschablone kann beispielsweise mittels eines 3D-Druckers oder mittels eines CAD/CAM-Verfahrens aus einem Rohling nach dem konstruierten 3D-Modell der Bohrschablone automatisch hergestellt werden.

Die dentale Kamera kann eine beliebige dreidimensionale dentale Kamera sein, die beispielsweise auf einem Streifenprojektionsverfahren oder einem konfokalen Vermessungsverfahren beruht. Die Zahnsituation kann die unmittelbare Umgebung der einzusetzenden Restauration oder auch einen größeren Bereich um die einzusetzende Restauration umfassen.

Die Vermessung mittels der dentalen Kamera kann aus unterschiedlichen Richtungen, wie einer okklusalen Richtung, einer lingualen Richtung, einer bukkalen Richtung oder einer labialen Richtung, erfolgen. Nach der Vermessung mittels der dentalen Kamera wird das 3D-Modell der Zahnsituation erzeugt. Anschließend wird das neuronale Netz für maschinelles Lernen auf das 3D-Modell der Zahnsituation angewendet. Nach der Analyse des 3D-Modells der Zahnsituation wird dann automatisch ein 3D-Modell der Bohrschablone erzeugt.

Ein neuronales Netzwerk (CNN) ist im Wikipedia Artikel "Convolutional Neural Network" mit dem Link https://de.wikipedia.org/wiki/Convolutional Neural Network ausführlich beschrieben.

Im Folgenden wird ein Verfahren unter Verwendung eines CNN erläutert.

Ein Convolutional Neural Network (CNN), zu Deutsch "faltendes neuronales Netzwerk", ist ein feed forward künstliches neuronales Netz. Es handelt sich um ein von biologischen Prozessen inspiriertes Konzept im Bereich des maschinellen Lernens. Convolutional Neural Networks finden Anwendung in zahlreichen modernen Technologien der künstlichen Intelligenz, vornehmlich bei der maschinellen Verarbeitung von Bild- oder Audiodaten.

Grundsätzlich besteht die Struktur eines klassischen CNN aus einem Convolutional Layer, gefolgt von einem Pooling Layer. Diese Einheit kann sich prinzipiell beliebig oft wiederholen, bei ausreichend Wiederholungen spricht man dann von Deep Convolutional Neural Networks, die in den Bereich Deep Learning fallen.

Die CNN lernen dadurch, dass freie Parameter bzw. Klassifikatoren der Convolution-Kernel pro Layer und deren Gewichtung bei der Verrechnung zum nächsten Layer gelernt werden.

Das 3D-Modell der Zahnsituation wird also als Eingabe des CNN bzw. Maschinen-Learning-Systems verwendet, welches unter Verwendung einer Sammlung einer Vielzahl von 3D-Modellen unterschiedlicher Zahnsituationen trainiert wurde.

In einem weiteren Schritt wird das 3D-Modell der Zahnsituation mittels des Maschinen-Learning-Systems analysiert und als Ausgabe wird ein 3D-Modell des jeweiligen Bauteils vorgeschlagen.

Das Maschinen-Learning-System kann aus einem oder mehreren CNN-Netzwerken bestehen.

Die Klassifikatoren bzw. Merkmale werden also automatisch festgelegt und bei der Analyse des Trainingssets verfeinert. Die automatisch ermittelten Klassifikatoren eines 3D-Modells einer Zahnsituation könnten beispielsweise eine Gesamtfläche einer Präparation oder der Verlauf des Präparationsrandes oder eine Kombination aus beidem sein.

Das Neuronale Netz kann beispielsweise aus mehreren Layern bestehen, wobei in einem ersten Layer einfache Klassifikatoren, wie Kanten, flache Oberflächen oder Bereiche gleicher Helligkeit automatisch identifiziert werden. In einem zweiten Layer werden die Klassifikatoren automatisch verfeinert. Die Klassifikatoren im zweiten Layer können beispielsweise die relative Anordnung der Kanten zueinander, die relative Richtung der Kanten oder der Verlauf der Kanten sein. In den weiteren Layern werden die Klassifikatoren immer weiter verfeinert und werden dadurch immer komplexer. Auf diese Art und Weise lernt das CNN- Netzwerk selbstständig, anhand des 3D-Modells der Zahnsituation und des Volumenmodells der Zahlensituation als Eingabeparameter automatisch ein passendes 3D-Modell der Bohrschablone zu erzeugen.

Der Vorteil eines Neuronalen Netzes besteht darin, dass die Parameterwerte der internen Convolution-Filter und die Weiterverarbeitung der Filterausgaben bei der Analyse des Trainingssets mitgelernt werden und deshalb keine weitere Benutzerspezifikation notwendig ist.

Ein weiterer Vorteil des Verfahrens liegt darin, dass die Konstruktion der Bohrschablone vollautomatisch mittels des Neuronalen Netzes erfolgt. Dadurch kann also das Bauteil unmittelbar nach der optischen Vermessung und/oder der Röntgen- bzw. MRT-Vermessung vollautomatisch konstruiert werden und nach einer Kontrolle durch den Zahnarzt vollautomatisch mittels eines 3D-Druckers oder einer CAD/CAM-Vorrichtung hergestellt werden, sodass die Bohrschablone innerhalb einer Sitzung zur Herstellung von Implantatbohrungen verwendet werden kann.

Ein weiterer Vorteil des Verfahrens ist, dass durch den Benutzer erzeugte fertig konstruierte Bohrschablonen im Trainingsdatensatz des CNN verwendet werden können und damit die Akzeptanzrate der Erstvorschläge der Bauteile und der Automatisierungsgrad der Konstruktion verbessert wird.

Im Folgenden wird ein mögliches Verfahren zum Training bzw. Parametrisierung des Maschinen-Learning-Systems bestehend aus einem oder mehreren CNN-Netzwerken erläutert. Im ersten Schritt wird eine große Anzahl bekannter 3D-Oberflächenmodelle und Volumenmodelle von Zahnsituationen analysiert. Dabei werden mögliche Eingabedaten bzw. Inputdaten generiert. Die Inputdaten werden so erzeugt, dass alle möglichen Freiheitsgrade in den Inputdaten vorhanden sind. Dies wird beispielsweise unter Verwendung einer Data-Augmentation erreicht. Dazu werden die 3D-Modelle der Zahnsituationen um die festgelegten Freiheitsgrade rotiert und/oder entlang der Freiheitsgrade skaliert.

Vorteilhafterweise kann das neuronale Netz anhand eines Trainingsdatensatzes trainiert werden, wobei der Trainingsdatensatz manuelle Änderungen von mehreren initialen 3D-Modellen von Bohrschablonen mindestens eines Benutzers beinhaltet, wobei die manuellen Änderungen beispielsweise unter Verwendung von CAD-Werkzeugen manuell durch den Benutzer bei der Konstruktion des 3D-Modells der jeweiligen Bohrschablone durchgeführt werden.

Dadurch lernt also das Neuronale Netz, die manuellen Änderungen eines bestimmten Benutzers auf einen initialen Vorschlag eines 3D-Modelles eines Bauteils anzuwenden, um die Konstruktion des Bauteils abzuschließen. Der initiale Vorschlag eines 3D-Modells des Bauteils kann beispielweise unter Verwendung einer Bibliothek mehrerer 3D-Modelle von Bauteilen ausgewählt werden. Bei der Anwendung des so trainierten Neuronalen Netzes auf ein unbekanntes 3D-Modell einer Zahnsituation werden dann automatisch für den jeweiligen Benutzer übliche Änderungen des Initialvorschlags durchgeführt. Die manuellen Änderungen des initialen 3D-Modells der Bohrschablone können beispielsweise die Anpassung des Implantattyps, die Anpassung der Implantatlage relativ zum Kieferknochen, die Anpassung der Länge einer Implantatbohrung, Anpassung eines Anschlages einer Führungsbohrung, die Anpassung der äußeren Abmessungen der Bohrschablone, die Änderung eines Lochdurchmessers der Führungsbohrung, das Einfügen von Auflagenkontrollfenstern und/oder das Anbringen von zusätzlichen Halteelementen sein.

Sogenannte Auflagenkontrollfenster dienen zur visuellen Überprüfung durch den Benutzer, ob die Bohrschablone richtig aufgesetzt ist und auf den Auflageflächen aufliegt.

Zusätzliche Halteelemente können beispielsweise Hinterschnitte oder Schrauben zur Fixierung der Bohrschablone relativ zum jeweiligen Kiefer sein.

Gemäß einer Weiterbildung kann das neuronale Netz zusätzlich auf ein initiales 3D-Modell der Bohrschablone angewendet werden. Vorteilhafterweise kann das neuronale Netz anhand eines Trainingsdatensatzes trainiert werden, wobei der Trainingsdatensatz mehrere 3D-Oberflächenmodelle und Volumenmodelle der Zahnsituationen und die entsprechenden 3D-Modelle der fertig konstruierten Bohrschablonen mindestens eines Benutzers beinhaltet.

Das Neuronale Netz lernt also sowohl aus den Vergleichsdaten zwischen einem initialen 3D-Modell und einem fertig konstruierten 3D-Modell der Bohrschablone, als auch aus den Anordnungen der anatomischen Strukturen, wie Zahnwurzeln, Zahnnerven und Kieferknochen innerhalb des Volumenmodells relativ zum fertig konstruierten 3D-Modell der Bohrschablone.

Vorteilhafterweise kann das neuronale Netz anhand eines Trainingsdatensatzes trainiert werden, wobei der Trainingsdatensatz mehrere initiale 3D-Modelle der Bohrschablonen und entsprechende fertig konstruierte 3D-Modelle der Bohrschablonen mindestens eines Benutzers beinhaltet.

Das Neuronale Netz lernt also aus den Vergleichsdaten zwischen einem initialen 3D-Modell der Bohrschablone und dem entsprechenden fertig konstruierten 3D-Modell der Bohrschablone.

Vorteilhafterweise kann der Trainingsdatensatz nur die Daten eines Benutzers oder einer Gruppe von erfahrenen Benutzern enthält.

Für jeden Benutzer bzw. für eine Gruppe erfahrener Benutzer gibt es also einen einzelnen Trainingsdatensatz.

Dadurch kann das Neuronale Netz also durch einen Trainingsdatensatz eines einzelnen Benutzers oder einer Gruppe von Benutzern trainiert werden. Die Benutzer für den Trainingsdatensatz können nach verschiedenen Kriterien, wie z.B. Berufserfahrung, ausgesucht werden. Für den Trainingsdatensatz können beispielsweise nur Daten von Benutzern verwendet werden, die mindestens drei Jahre Berufserfahrung haben oder mindestens 100 Fälle bei der Konstruktion von Bohrschablonen ausgeführt haben.

Der Benutzer kann vor der Durchführung des vorliegenden Verfahrens die Art der jeweiligen Bohrschablone und die Positionen der durchzuführenden Implantatbohrung manuell, beispielsweise mittels eines Auswahlmenüs, festlegen. Die Art der Bohrschablone kann beispielsweise eine Bohrschablone ohne Bohrhülsen, eine Bohrschablone mit Bohrhülsen, eine Bohrschablone mit Schrauben zur Befestigung am Kieferknochen oder eine Bohrschablone mit einer Auflagefläche zur Verankerung an den Nachbarzähnen sein.

Vorteilhafterweise kann das neuronale Netz nach dem Trainieren anhand des Trainingsdatensatzes unverändert bleiben oder dem Trainingsdatensatz neue Daten hinzugefügt werden, so dass das neuronale Netz anhand des erweiterten Trainingsdatensatzes weiter trainiert wird.

Insbesondere für unerfahrene Benutzer ist es am Anfang vorteilhaft, ein trainiertes Neuronale Netz nicht zu verändern. Für erfahrene Benutzer ist es vorteilhafter, ihre eigenen Daten der Konstruktion der Bohrschablone einem Trainingsdatensatz hinzuzufügen, sodass das Neuronale Netz kontinuierlich weiter trainiert wird und die Anforderungen des jeweiligen Benutzers mit der Zeit besser erfüllt. Dem erweiterten Trainingsdatensatz werden also weitere Trainingsdaten hinzugefügt.

Vorteilhafterweise kann das 3D-Oberflächenmodell der Zahnsituation mindestens einen Bereich für eine Implantatversorgung mit einzusetzenden Implantaten, mindestens einen Nachbarzahn und/oder eine implantatgetragene Mesostruktur, wie ein Abutment, aufweisen.

Das 3D-Modell der jeweiligen Bohrschablone wird also in Abhängigkeit von den Strukturen, die im 3D-Modell der Zahnsituation enthalten sind, automatisch durch das Neuronale Netz konstruiert.

Vorteilhafterweise kann das neuronale Netz für die herzustellende Bohrschablone automatisch ein passendes Material, ein Fertigungsverfahren und/oder ein Einsetzungsverfahren festlegen.

Das passende Material kann beispielsweise ein spezieller Kunststoff oder eine Kombination aus verschiedenen Kunststoffen sein. Das Fertigungsverfahren kann beispielsweise die Herstellung mittels eines 3D-Druckers oder CAD/CAM-Verfahren sein. Das Einsetzungsverfahren kann beispielsweise das Befestigen der Bohrschablone mittels Schrauben oder durch das Aufsetzen der Bohrschablone auf den Nachbarzähnen sein.

Vorteilhafterweise kann der Trainingsdatensatz zusätzlich ein Material der Bohrschablone, ein Fertigungsverfahren und/oder ein Einsetzungsverfahren der Bohrschablone beinhalten.

Dadurch enthält der Trainingsdatensatz weitere wesentliche Informationen der Bohrschablone, sodass das trainierte neuronale Netz eine passende Bohrschablone konstruieren kann, die den Anforderungen des jeweiligen Benutzers entspricht.

Vorteilhafterweise kann der Trainingsdatensatz bei einer Konstruktion einer Bohrschablone mit Bohrhülsen zusätzlich eine Lage, eine Form und/oder eine Art der Bohrhülsen beinhalten.

Dadurch enthält der Trainingsdatensatz wesentliche Informationen bezüglich der Bohrhülsen, so dass das trainierte neuronale Netz die Bohrhülsen, wie vom Benutzer gewünscht, auswählt und positioniert.

Vorteilhafterweise kann der Trainingsdatensatz zusätzlich eine Form und/oder Lage von mindestens einer Stützstruktur bzw. Haltestruktur der herzustellenden Bohrschablone beinhalten.

Dadurch enthält der Trainingsdatensatz wesentliche Informationen bezüglich einer Stützstruktur bzw. Haltestruktur. Die Stützstruktur kann beispielsweise eine Auflagefläche zum Aufsetzen auf den Nachbarzähnen des Implantationsbereichs sein. Die Stützstruktur kann beispielsweise in Form eines Abdrucks der Nachbarzähne geformt sein, um eine präzise Positionierung der Bohrschablone relativ zum Kiefer zu gewährleisten.

Vorteilhafterweise kann der Trainingsdatensatz zusätzlich eine Form und/oder Lage einer Verankerungsstruktur für die herzustellende Bohrschablone beinhalten.

Dadurch enthält der Trainingsdatensatz wesentliche Informationen bezüglich der Verankerungsstruktur, wie Schrauben, die die Bohrschablone am Kieferknochen fixieren, so dass das neuronale Netz automatisch die Verankerungsstruktur auswählen und relativ zum Kieferknochen positionieren kann.

Vorteilhafterweise kann der Trainingsdatensatz zusätzlich Hinterschnitte zur Verankerung der Bohrschablone und/oder zusätzliche Löcher mit Schrauben zur Fixierung der Bohrschablone beinhalten.

Dadurch enthält der Trainingsdatensatz zusätzliche Informationen bezüglich mehrerer Hinterschnitte zur Verankerung der Bohrschablone und/oder bezüglich zusätzlicher Löcher für Schrauben zur Fixierung der Bohrschablone am Kieferknochen, so dass das neuronale Netz selbstständig die Hinterschnitte der Bohrschablone bzw. die zusätzlichen Löcher für die Schrauben konstruieren kann. Die Hinterschnitte an der Bohrschablone werden so konstruiert, dass die Hinterschnitte beispielsweise um die Backenzähne greifen und dadurch die Bohrschablone beim Aufsetzen auf die Zähne fixiert wird.

Vorteilhafterweise kann der Trainingsdatensatz zusätzlich mindestens eine Führungsbohrung der Bohrschablone für eine herzustellende Implantatbohrung und/oder einen Auflagebereich zur Abstützung bzw. Fixierung der Bohrschablone und/oder eine Schraube zur Fixierung der Bohrschablone beinhalten.

Dadurch enthält der Trainingsdatensatz zusätzliche Informationen bezüglich der Führungsbohrung bzw. des Auflagebereichs, so dass das neuronale Netz automatisch die Lage und Ausrichtung der Führungsbohrung auf der Bohrschablone festlegen kann bzw. die Form und Anordnung des Auflagebereichs auf der Bohrschablone festlegen kann. Die Führungsbohrung der Bohrschablone dient dabei zur Führung eines Implantatbohrers bei der Durchführung einer Implantatbohrung. Die Führungsbohrung kann dabei mit einer Bohrhülse versehen sein.

Vorteilhafterweise kann der Trainingsdatensatz zusätzlich anatomische Strukturen im Volumenmodell beinhalten, nämlich Kieferknochen, Zahnwurzeln und/oder Zahnnerven, die bei der Konstruktion einer Implantatbohrung berücksichtigt werden.

Dadurch enthält der Trainingsdatensatz zusätzliche Informationen bezüglich wesentlicher anatomischer Strukturen für die Planung einer Implantatbohrung, so dass das neuronale Netz automatisch die Konstruktion einer Implantatbohrung unter Berücksichtigung dieser Strukturen durchführen kann.

Vorteilhafterweise kann das neuronale Netz eine ethnische Gruppe und/oder einen Charaktertyp des Patienten berücksichtigen.

Dadurch können charakteristische anatomische Merkmale einer ethnischen Gruppe bei der Konstruktion der Bohrschablone berücksichtigt werden. Die charakteristischen Merkmale einer ethnischen Gruppe können kann beispielsweise eine charakteristische Zahnstellung sein.

Ein weiterer Gegenstand der folgenden Erfindung ist eine Vorrichtung zur Datenverarbeitung, umfassend mit zur Ausführung des erfinderischen Verfahrens. Die Vorrichtung zur Datenverarbeitung kann dabei einen Computerchip aufweisen und beispielsweise ein Computer oder ein Smartphone sein.

### Kurzbeschreibung der Zeichnungen

Die Erfindung wird anhand der Zeichnungen erläutert. Es zeigt, die
- Fig. 1: eine Skizze zur Verdeutlichung des Verfahrens zur Konstruktion der Bohrschablone, die
- Fig. 2: eine Skizze der Bohrschablone mit einer Bohrhülse und einer Schraube zur Fixierung;
- Fig. 3: eine Skizze der Bohrschablone mit Hinterschnitten zur Verankerung an den Zähnen
- Fig. 4: eine Skizze der Änderungen durch einen Benutzer.

### Ausführungsbeispiele

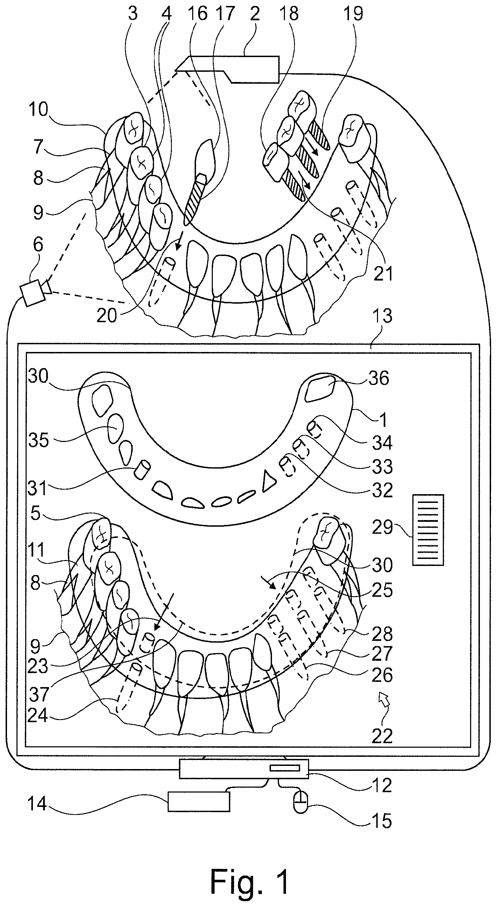

Die Fig. 1 zeigt eine Skizze zur Verdeutlichung des Verfahrens zur Konstruktion einer Bohrschablone 1, wobei mittels einer dentalen Kamera 2 eine Zahnsituation 3, umfassend Zähne 4 vermessen wird und ein 3D-Oberflächenmodell 5 der Zahnsituation 3 erzeugt wird. Zusätzlich wird mittels einer CT-Röntgenvorrichtung 6 beziehungsweise einer MRT-Vorrichtung ein Volumen 7 umfassend Zahnwurzeln 8, Zahnnerven 9 und einen Kieferknochen 10 vermessen und ein Volumenmodell 11 der Zahnsituation 3 erzeugt. Die Bilddaten der dentalen Kamera 2 werden an einen Computer 12 weitergeleitet, wobei die Bilddaten der CT-Röntgenvorrichtung 6 beziehungsweise der MRT-Vorrichtung ebenfalls an den Computer 12 weitergeleitet werden. Aus diesen Bilddaten werden mittels des Computers 12 das 3D-Oberflächenmodell 5 und das Volumenmodell 11 erzeugt, die mittels einer Anzeigevorrichtung 13, wie eines Monitors, angezeigt werden. An den Computer 12 sind Eingabemittel, wie eine Tastatur 14 und eine Maus 15, angeschlossen, um den Benutzer eine Navigation mittels eines Cursors 22 innerhalb einer grafischen Darstellung des 3D-Oberflächenmodells 5 und des Volumenmodells 11 zu ermöglichen. Die Zahnsituation 3 weist einen fehlenden Eckzahn und drei fehlende Backenzähne auf. Eine implantatgetragene Vollkrone 16 mit einem Implantat 17 soll also den fehlenden Eckzahn ersetzen, wobei eine implantatgetragene Brücke 18 aus drei künstlichen Backenzähnen und den dazugehörigen Implantaten 19 die fehlenden Backenzähne ersetzen soll, wie durch die Pfeile 20 und 21 angedeutet. In einem ersten Schritt kann der Benutzer manuell mittels des Cursors 22 in etwa eine erste Position 23 einer ersten Implantatbohrung 24 und in etwa eine zweite Position 25 der einzusetzenden Brücke 18 und damit einer zweiten Implantatbohrung 26, einer dritten Implantatbohrung 27 und einer vierten Implantatbohrung 28 festlegen. In einem weiteren Schritt kann mittels des Cursors 22 unter Verwendung eines Auswahlmenüs 29 die Art der zu konstruierenden Bohrschablone ausgewählt werden, nämlich eine Bohrschablone mit oder ohne Bohrhülsen, eine Bohrschablone mit einer Verankerung am Kiefer durch Schrauben oder durch eine Auflagefläche für die Nachbarzähne. In einem weiteren Schritt wird mittels des neuronalen Netzes, welches anhand eines Trainingsdatensatzes für den jeweiligen Benutzer und für die jeweilige Art der Bohrschablone trainiert wurde, automatisch ein 3D-Modell 30 der Bohrschablone erzeugt. Das erzeugte 3D-Modell der Bohrschablone 1 weist eine erste Führungsbohrung 31 für die erste Implantatbohrung 24, eine zweite Führungsbohrung 32 für die zweite Implantatbohrung 26, eine dritte Führungsbohrung 33 für die dritte Implantatbohrung 27 und eine vierte Führungsbohrung 34 für die vierte Implantatbohrung 28 auf. Darüber hinaus weist das 3D-Modell 30 der Bohrschablone 1 eine erste Auflagefläche 35 und eine zweite Auflagefläche 36 zur Verankerung der Bohrschablone am Kiefer, wobei die Auflageflächen 35 und 36 Teile der negativen Abdrücke der Zähne 4 der Zahnsituation 3 umfassen können. Die Führungsbohrungen 31, 32, 33 und 34 können Bohrhülsen zur Führung eines entsprechenden Implantatbohrers aufweisen. Das neuronale Netz berücksichtigt bei der Festlegung der Lage und Ausrichtung der einzelnen Implantatbohrungen 24, 26, 27 und 28 wesentliche anatomische Strukturen, wie Zahnwurzeln 8, Zahnnerven 9 und den Kieferknochen, aus dem Volumenmodell 11. Das neuronale Netz kann beispielsweise lernen, dass die Implantatbohrung 24 den Nerv 9 nicht verletzen darf. Das neuronale Netz kann den Durchmesser und die Länge der Implantatbohrungen 24, 26, 27 und 28 automatisch festlegen, wobei mechanische Belastungen der einzusetzenden Vollkrone 16 und der Brücke 18 berücksichtigt werden können. In der grafischen Darstellung auf der Anzeigevorrichtung 13 ist das 3D-Modell 30 der Bohrschablone 1 im eingesetzten Zustand relativ zum 3D-Oberflächenmodell 5 und dem Volumenmodell 11 durch eine gestrichelte Linie 37 dargestellt. Unter Verwendung der festgelegten Lage, Durchmesser und Länge der Implantatbohrungen 24, 26, 27 und 28 kann das neuronale Netz die Lage und die Durchmesser der entsprechenden Führungsbohrungen 31, 32, 33 und 34 der Bohrschablone 1 festlegen. Das fertig konstruierte 3D-Modell 30 der Bohrschablone 1 kann anschließend mittels eines automatisierten Verfahrens, wie eines 3D-Druckers oder eines CAD/CAM-Verfahrens, automatisch hergestellt werden.

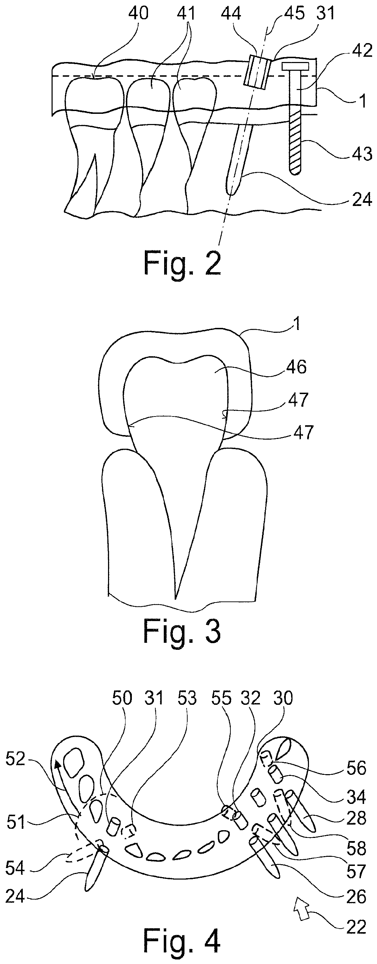

Die Fig.2 zeigt eine Skizze der Bohrschablone 1 zur Durchführung der ersten Implantatbohrung 24 aus Fig. 1, wobei die Bohrschablone 1 mittels einer Auflagefläche 40 auf den Nachbarzähnen 41 und mittels einer Schraube 42 am Kiefer 43 verankert wird. Die Führungsbohrung 31 weist eine Bohrhülse 44 zur Führung eines Implantatbohrers auf. Die Mittelachse 45 der Implantatbohrung 24 entspricht also der Mittelachse der Bohrhülse 44. Die Auflagefläche 40 kann auch als negativer Abdruck der Zähne 41 geformt sein.

Die Fig. 3 zeigt eine Skizze eines Querschnittes der Schablone 1, die auf den Backenzähnen 46 aufgesetzt ist. Die Bohrschablone weist dabei zwei Hinterschnitte 47 im unteren Bereich auf, so dass beim Aufsetzen der aus elastischem Kunststoff hergestellten Bohrschablone 1 auf die Backenzähne 46 eine mechanische Verankerung der Bohrschablone 1 zu den Zähnen 46 hergestellt wird.

Die Fig. 4 zeigt eine Skizze des fertig konstruierten 3D-Modells 30 der Bohrschablone 1 aus Fig. 1, wobei ausgehend von einem initialen Vorschlag 50 eines 3D-Modells der Bohrschablone, der durch eine gestrichelte Linie dargestellt ist, der Benutzer durch manuelle Änderungen mittels des Cursors 22 den initialen Vorschlag 50 geändert bzw. angepasst hat, um das fertig konstruierte 3D-Modell 30 zu erhalten. Der initiale Vorschlag 50 weist dabei äußere Abmessungen 51 auf, die durch den Benutzer vergrößert wurden, wie durch den Pfeil 52 angedeutet ist, um die äußeren Abmessungen des 3D-Modells 30 zu erhalten. Der initiale Vorschlag 50 weist eine erste initiale Führungsbohrung 53 auf, um eine erste initiale Implantatbohrung 54 durchzuführen. Der Benutzer hat die Lage und einen Anschlag der initialen Führungsbohrung 53 verändert, um die fertig konstruierte erste Führungsbohrung 31 zu erhalten, wobei durch die Veränderung der Lage auch die Lage der Implantatbohrung verändert wurde und durch einen kleineren Anschlag die Länge der Implantatbohrung vergrößert wurde. Ein Anschlag dient bei einer Führungsbohrung zur Begrenzung des Implantatbohrers bei Bohrung der Implantatbohrung, so dass durch den Anschlag die Länge der Implantatbohrung bestimmt wird. Diese Änderungen werden also durchgeführt, um die fertig konstruierte Implantatbohrung 24 zu erhalten. Entsprechend werden auch die übrigen initialen Führungsbohrungen 55 und 56 manuell durch den Benutzer angepasst, um die fertig konstruierten Führungsbohrungen 32 und 34 zu erhalten, wobei auch die initialen Implantatbohrungen 57 und 58 bezüglich der Lage und der Länge angepasst werden, um die fertig konstruierten Implantatbohrungen 26 und 28 zu erhalten. Ein Trainingsdatensatz des neuronalen Netzes kann also solche manuellen Änderungen des jeweiligen Benutzers umfassen, um das neuronale Netz entsprechend zu trainieren.

### Bezugszeichen

- 1: Bohrschablone
- 2: Dentale Kamera
- 3: Zahnsituation
- 4: Zähne
- 5: 3D-Oberflächenmodell
- 6: CT-Röntgenvorrichtung
- 7: Volumen
- 8: Zahnwurzeln
- 9: Zahnnerven
- 10: Kieferknochen
- 11: Volumenmodell
- 12: Computer
- 13: Anzeigevorrichtung
- 14: Tastatur
- 15: Maus
- 16: Implantatgetragene Vollkrone
- 17: Implantat
- 18: Implantatgetragene Brücke
- 19: Implantat
- 20: Pfeil
- 21: Pfeil
- 22: Cursor
- 23: Erste Position
- 24: Erste Implantatbohrung
- 25: Zweite Position
- 26: Zweite Implantatbohrung
- 27: Dritte Implantatbohrung
- 28: Vierte Implantatbohrung
- 29: Auswahlmenü
- 30: 3D-Modell
- 31: Führungsbohrung
- 32: Führungsbohrung
- 33: Führungsbohrung
- 34: Führungsbohrung
- 35: Auflagefläche
- 36: Auflagefläche
- 40: Auflagefläche
- 41: Nachbarzähne
- 42: Schraube
- 43: Kiefer
- 44: Bohrhülse
- 45: Mittelachse
- 46: Backenzähne
- 47: Hinterschnitte
- 50: initialer Vorschlag
- 51: äußere Abmessungen
- 52: Pfeil
- 53: initiale Führungsbohrung
- 54: initiale Implantatbohrung
- 55: initiale Führungsbohrung
- 56: initiale Führungsbohrung
- 57: initiale Implantatbohrung
- 58: initiale Implantatbohrung

## Patentansprüche

1. Verfahren zur Konstruktion einer Bohrschablone (1), wobei
mittels einer 3D-Oberflächenvermessungsvorrichtung eine Zahnsituation (3) vermessen wird und ein 3D-Oberflächenmodell (5) der Zahnsituation (3) erzeugt wird und
mittels einer Röntgenvorrichtung (6) oder einer MRT-Vorrichtung die Zahnsituation (3) vermessen wird und ein Volumenmodell (11) der Zahnsituation (3) erzeugt wird,
**dadurch gekennzeichnet, dass** ein künstliches neuronales Netz für maschinelles Lernen (engl. Convolutional Neural Network; CNN)
auf das 3D-Oberflächenmodell (5) der Zahnsituation (3) und
auf das Volumenmodell (11) der Zahnsituation (3) angewendet wird und automatisch ein fertig konstruiertes 3D-Modell (30) der Bohrschablone (1) erzeugt.

2. Verfahren nach Anspruch 1, **dadurch gekennzeichnet, dass** das neuronale Netz zusätzlich auf ein initiales 3D-Modell (50) der Bohrschablone (1) angewendet wird.

3. Verfahren nach Anspruch 2, **dadurch gekennzeichnet, dass** das neuronale Netz anhand eines Trainingsdatensatzes trainiert wird, wobei der Trainingsdatensatz manuelle Änderungen (52) von mehreren initialen 3D-Modellen (50) von Bohrschablonen (1) mindestens eines Benutzers beinhaltet, wobei die manuellen Änderungen (52) durch den Benutzer bei der Konstruktion des 3D-Modells (30) der jeweiligen Bohrschablone (1) durchgeführt werden.

4. Verfahren nach Anspruch 1 oder 2, **dadurch gekennzeichnet, dass** das neuronale Netz anhand eines Trainingsdatensatzes trainiert wird, wobei der Trainingsdatensatz mehrere 3D-Oberflächenmodelle (5) und Volumenmodelle (11) der Zahnsituationen (3) und die entsprechenden 3D-Modelle (30) der fertig konstruierten Bohrschablonen (1) mindestens eines Benutzers beinhaltet.

5. Verfahren nach Anspruch 2, **dadurch gekennzeichnet, dass** das neuronale Netz anhand eines Trainingsdatensatzes trainiert wird, wobei der Trainingsdatensatz mehrere initiale 3D-Modelle (50) der Bohrschablonen (1) und entsprechende fertig konstruierte 3D-Modelle (30) der Bohrschablonen (1) mindestens eines Benutzers beinhaltet.

6. Verfahren nach einem der Ansprüche 3 bis 5, **dadurch gekennzeichnet, dass** der Trainingsdatensatz nur die Daten eines Benutzers oder einer Gruppe von erfahrenen Benutzern enthält.

7. Verfahren nach einem der Ansprüche 3 bis 6, **dadurch gekennzeichnet, dass** das neuronale Netz nach dem Trainieren anhand des Trainingsdatensatzes unverändert bleibt.

8. Verfahren nach einem der Ansprüche 3 bis 6, **dadurch gekennzeichnet, dass** dem Trainingsdatensatz neue Daten hinzugefügt werden, so dass das neuronale Netz anhand des erweiterten Trainingsdatensatzes weiter trainiert wird.

9. Verfahren nach Anspruch 7 oder 8, **dadurch gekennzeichnet, dass** das 3D-Oberflächenmodell (5) der Zahnsituation (3) mindestens einen Bereich für eine Implantatversorgung mit einzusetzenden Implantaten (17, 19), mindestens einen Nachbarzahn (4) und/oder eine implantatgetragene Mesostruktur, wie ein Abutment, aufweist.

10. Verfahren nach einem der Ansprüche 1 bis 9, **dadurch gekennzeichnet, dass** das neuronale Netz für die herzustellende Bohrschablone (1) automatisch ein Material, ein Fertigungsverfahren und/oder ein Einsetzungsverfahren festlegt.

11. Verfahren nach einem der Ansprüche 3 bis 10, **dadurch gekennzeichnet, dass** der Trainingsdatensatz zusätzlich ein Material der Bohrschablone (1), ein Fertigungsverfahren und/oder ein Einsetzungsverfahren der Bohrschablone (1) beinhaltet.

12. Verfahren nach Anspruch 3 bis 11, **dadurch gekennzeichnet, dass** der Trainingsdatensatz bei einer Konstruktion einer Bohrschablone (1) mit Bohrhülsen (44) zusätzlich eine Lage, eine Form und/oder eine Art der Bohrhülsen (44) beinhaltet.

13. Verfahren nach einem der Ansprüche 3 bis 12, **dadurch gekennzeichnet, dass** der Trainingsdatensatz zusätzlich eine Form und/oder Lage von mindestens einer Stützstruktur bzw. Haltestruktur der herzustellenden Bohrschablone (1) beinhaltet.

14. Vorrichtung zur Datenverarbeitung, umfassend Mittel zur Ausführung des Verfahrens nach einem der Ansprüche 1 bis 13.

15. Computerprogramm, umfassend Befehle, die bei der Ausführung des Computerprogramms durch einen Computer diesen veranlassen, das Verfahren nach einem der Ansprüche 1 bis 13 auszuführen.

16. Computerlesbares Speichermedium, umfassend Befehle, die bei der Ausführung durch einen Computer diesen veranlassen, das Verfahren nach einem der Ansprüche 1 bis 13 auszuführen.

## Claims

1. A method for designing a drilling template (1), wherein a dental situation (3) is measured using a 3D surface measurement device and a 3D surface model (5) of the dental situation (3) is generated and the dental situation (3) is measured using an X-ray device (6) or an MRI device and a volume model (11) of the dental situation (3) is generated, **characterised in that** an artificial neural network for machine learning (convolutional neural network; CNN) is applied to the 3D surface model (5) of the dental situation (3) and to the volume model (11) of the dental situation (3) and a fully designed 3D model (30) of the drilling template (1) is automatically generated.

2. The method according to claim 1, **characterised in that** the neural network is additionally applied to an initial 3D model (50) of the drilling template (1).

3. The method according to claim 2, **characterised in that** the neural network is trained using a training data set, the training data set including manual changes (52) from a plurality of initial 3D models (50) of drilling templates (1) of at least one user, the manual changes (52) being carried out by the user during the design of the 3D model (30) of a particular drilling template (1).

4. The method according to claim 1 or 2, **characterised in that** the neural network is trained using a training data set, the training data set comprising a plurality of 3D surface models (5) and volume models (11) of the dental situations (3) and the corresponding 3D models (30) of the fully designed drilling templates (1) of at least one user.

5. The method according to claim 2, **characterised in that** the neural network is trained on the basis of a training data set, the training data set containing a plurality of initial 3D models (50) of the drilling templates (1) and corresponding fully designed 3D models (30) of the drilling templates (1) of at least one user.

6. The method according to any one of claims 3 to 5, **characterised in that** the training data set only contains the data of one user or a group of experienced users.

7. The method according to any one of claims 3 to 6, **characterised in that** the neural network remains unchanged after the training on the basis of the training data set.

8. The method according to any one of claims 3 to 6, **characterised in that** new data is added to the training data set, so that the neural network is trained further on the basis of the expanded training data set.

9. The method according to claim 7 or 8, **characterised in that** the 3D surface model (5) of the dental situation (3) has at least one area for implant treatment with implants (17, 19) to be inserted, at least one neighbouring tooth (4) and/or an implant-borne mesostructure, such as an abutment.

10. The method according to any one of claims 1 to 9, **characterised in that** the neural network for the drilling template (1) to be produced automatically defines a material, a manufacturing method and/or an insertion method.

11. The method according to any one of claims 3 to 10, **characterised in that** the training data set additionally includes a material of the drilling template (1), a manufacturing method and/or an insertion method of the drilling template (1).

12. The method according to claims 3 to 11, **characterised in that**, when designing a drilling template (1) with drilling sleeves (44), the training data set additionally contains a position, a shape and/or a type of drilling sleeves (44).

13. The method according to any one of claims 3 to 12, **characterised in that** the training data set additionally includes a shape and/or position of at least one support structure or holding structure of the drilling template (1) to be produced.

14. A device for data processing, comprising means for executing the method according to any one of claims 1 to 13.

15. A computer program, comprising commands, which, when the computer program is run by a computer, cause said computer to execute the method according to any one of claims 1 to 13.

16. A computer-readable storage medium, comprising commands, which, when run by a computer, cause said computer to execute the method according to any one of claims 1 to 13.

## Revendications

1. Procédé de réalisation d'un gabarit de perçage (1), une situation dentaire (3) étant mesurée, à l'aide d"un appareil de mesure de surface 3D et un modèle de surface 3D (5) de la situation dentaire (3) étant généré et la situation dentaire (3) étant mesurée à l'aide d'un appareil à rayons X (6) ou d'un dispositif d'IRM et un modèle de volume (11) de la situation dentaire (3) étant généré, **caractérisé en ce qu'**un réseau neuronal artificiel pour l'apprentissage automatique (en anglais : Convolutional Neural Network : CNN) est appliqué au modèle de surface 3D (5) de la situation dentaire (3) et au modèle de volume (11) de la situation dentaire (3) et génère automatiquement un modèle 3D (30) entièrement réalisé du gabarit de perçage (1).

2. Procédé selon la revendication 1, **caractérisé en ce que** le réseau neuronal est en outre appliqué à un modèle 3D (50) initial du gabarit de perçage (1).

3. Procédé selon la revendication 2, **caractérisé en ce que** le réseau neuronal est entraîné à l'aide d'un ensemble de données d'apprentissage, l'ensemble de données d'apprentissage comprenant des changements manuels (52) de plusieurs modèles 3D (50) initiaux de gabarits de perçage (1) d'au moins un utilisateur, les changements manuels (52) étant réalisés par l'utilisateur lors de la réalisation du modèle 3D (30) du gabarit de perçage (1) respectif.

4. Procédé selon la revendication 1 ou 2, **caractérisé en ce que** le réseau neuronal est entraîné à l'aide d'un ensemble de données d'apprentissage, l'ensemble de données d'apprentissage comprenant plusieurs modèles de surface 3D (5) et modèles de volume (11) des situations dentaires (3) et les modèles 3D (30) correspondants des gabarits de perçage (1) entièrement réalisés d'au moins un utilisateur.

5. Procédé selon la revendication 2, **caractérisé en ce que** le réseau neuronal est entraîné à l'aide d'un ensemble de données d'apprentissage, l'ensemble de données d'apprentissage comprenant au moins plusieurs modèles 3D (50) initiaux des gabarits de perçage (1) et modèles 3D (30) entièrement réalisés correspondants des gabarits de perçage (1) d'au moins un utilisateur.

6. Procédé selon l'une quelconque des revendications 3 à 5, **caractérisé en ce que** l'ensemble de données d'apprentissage ne contient que les données d'un utilisateur ou d'un groupe d'utilisateurs expérimentés.

7. Procédé selon l'une quelconque des revendications 3 à 6, **caractérisé en ce que** le réseau neuronal reste inchangé après l'entraînement à l'aide de l'ensemble de données d'apprentissage.

8. Procédé selon l'une quelconque des revendications 3 à 6, **caractérisé en ce que** de nouvelles données sont ajoutées à l'ensemble de données d'apprentissage, de sorte que le réseau neuronal continue d'être entraîné à l'aide de l'ensemble de données d'apprentissage étendu.

9. Procédé selon la revendication 7 ou 8, **caractérisé en ce que** le modèle de surface 3D (5) de la situation dentaire (3) présente au moins une zone de traitement implantaire avec des implants (17, 19) à insérer, au moins une dent voisine (4) et/ou une mésostructure implanto-portée, comme un pilier.

10. Procédé selon l'une quelconque des revendications 1 à 9, **caractérisé en ce que** le réseau neuronal pour le gabarit de perçage (1) à produire définit automatiquement un matériau, un procédé de fabrication et/ou un procédé d'insertion.

11. Procédé selon l'une quelconque des revendications 3 à 10, **caractérisé en ce que** l'ensemble de données d'apprentissage comprend en outre un matériau du gabarit de perçage (1), un procédé de fabrication et/ou un procédé d'insertion du gabarit de perçage (1).

12. Procédé selon les revendications 3 à 11, **caractérisé en ce que** l''ensemble de données d'apprentissage contient en outre une position, une forme et/ou un type de douilles de perçage (44) lors de la construction d'un gabarit de perçage (1) avec des douilles de perçage (44).

13. Procédé selon l'une quelconque des revendications 3 à 12, **caractérisé en ce que** l'ensemble de données d'apprentissage comprend en outre une forme et/ou une position d'au moins une structure de support ou structure de maintien du gabarit de perçage (1) à produire.

14. Dispositif de traitement de données, comprenant des moyens pour exécuter le procédé selon l'une quelconque des revendications 1 à 13.

15. Programme informatique, comprenant des instructions qui, lorsque le programme informatique est exécuté par un ordinateur, amènent ce dernier à exécuter le procédé selon l'une quelconque des revendications 1 à 13.

16. Support d'enregistrement lisible par ordinateur comprenant des instructions qui, lorsqu'elles sont exécutées par un ordinateur, amènent ce dernier à exécuter le procédé selon l'une quelconque des revendications 1 à 13.
